# FASCICULE DE BREVET EUROPEEN

(11) **EP 3 229 818 B1**
(45) Date de publication et mention de la délivrance du brevet: **24.07.2019**
(21) Numéro de dépôt: 15823635.6
(22) Date de dépôt: 11.12.2015
(51) Int. Cl.: A61K 35/644, A61P 39/00

(54) **UTILISATION D'UN EXTRAIT PARTICULIER DE PROPOLIS POUR LUTTER CONTRE LES EFFETS SECONDAIRES DES CHIMIOTHERAPIES**
VERWENDUNG EINES PARTIKELEXTRAKTES AUS PROPOLIS ZUR BEKÄMPFUNG DER NEBENWIRKUNGEN EINER CHEMOTHERAPIE
USE OF A PARTICULAR EXTRACT OF PROPOLIS FOR COMBATING THE SIDE EFFECTS OF CHEMOTHERAPY

(30) Priorité: 11.12.2014 FR 1462244
(43) Date de publication de la demande: 18.10.2017
(73) Titulaire: Pollenergie, 47450 Saint-Hilaire-de-Lusignan (FR)
(72) Inventeur: CARDINAULT, Nicolas, 47000 Agen (FR)
(74) Mandataire: Aquinov
(86) Numéro de dépôt international: PCT/FR2015/053453
(87) Numéro de publication internationale: WO 2016/092235

(56) Documents cités:
- M LAHOUEL ET AL: "Effet protecteur des flavonoïdes contre la toxicité de la vinblastine, du cyclophosphamide et du paracétamol par inhibition de la peroxydation lipidique et augmentation du glutathion hépatique", PATHOLOGIE BIOLOGIE, vol. 52, no. 6, 1 juillet 2004 (2004-07-01), pages 314-322, XP055082656, ISSN: 0369-8114, DOI: 10.1016/j.patbio.2004.01.001
- SUZUKI I ET AL: "Antitumor and anticytopenic effects of aqueous extracts of propolis in combination with chemotherapeutic agents", CANCER BIOTHERAPY & RADIOPHARMACEUTICALS, MARY ANN LIEBERT, US, vol. 17, no. 5, 1 janvier 2002 (2002-01-01), pages 553-562, XP002505357, ISSN: 1084-9785, DOI: 10.1089/108497802760804781
- ORSOLIC N ET AL: "Antitumor, hematostimulative and radioprotective action of water-soluble derivative of propolis (WSDP)", BIOMEDICINE AND PHARMACOTHERAPY, ELSEVIER, FR, vol. 59, no. 10, 1 décembre 2005 (2005-12-01), pages 561-570, XP027640259, ISSN: 0753-3322 [extrait le 2005-12-01]
- MARCUCCI M C: "Propolis:chemical composition, biological properties and therapeutic activity", APIDOLOGIE, ARBEITSGEMEINSCHAFT DER INSTITUTE FUER BIENENFORSCHUNG, CELLE, DE, vol. 26, 1 janvier 1995 (1995-01-01), pages 83-99, XP002524986, ISSN: 0044-8435, DOI: 10.1051/APIDO:19950202

## Description

La présente invention concerne la prévention et la lutte contre les effets secondaires des chimiothérapies.

La chimiothérapie est l'un des principaux traitements du cancer. Il s'agit d'un traitement agressif qui permet de s'attaquer aux cellules cancéreuses disséminées dans l'organisme. Toutefois, l'agression des cellules cancéreuses n'est pas suffisamment ciblée et entraîne très souvent des effets secondaires désagréables et néfastes pour le patient, tels qu'une diminution des globules rouges, des globules blancs et des plaquettes, des nausées et vomissements, un état de fatigue important, la chute des cheveux et des ongles, des défaillances hépatiques, rénales et / ou cardiaque, etc.

Il est donc nécessaire de trouver une solution efficace pouvant prévenir et limiter le plus possible les effets secondaires qui découlent des traitements par chimiothérapie. Actuellement, il n'existe pas de solution satisfaisante. Dans le cas d'une diminution trop importante des globules rouges, les oncologues doivent retarder le protocole de chimiothérapie initialement prévu pour laisser à l'organisme le temps de re-synthétiser ses globules rouges ou doivent accélérer cette synthèse en injectant un facteur de croissance, l'EPO qui présente l'inconvénient de favoriser la croissance des cellules cancéreuses également.

Une solution a été récemment proposée pour préparer l'organisme à être agressé par les agents de chimiothérapie, qui consiste à administrer de la propolis.

La propolis est un produit fabriqué par les abeilles à partir de substances résineuses, gommeuses et balsamiques, récoltées sur les bourgeons de certains arbres et arbustes auxquelles elles y incorporent des sécrétions salivaires.

Néanmoins, tous les extraits de propolis ne sont pas efficaces et l'objectif de la présente demande est de proposer une alternative efficace pour prévenir les effets secondaires des agents de chimiothérapie en utilisant un extrait de propolis présentant des caractéristiques particulières qui lui confèrent cette efficacité et qui n'interfère pas avec l'efficacité de l'agent de chimiothérapie.

Pour y répondre, l'invention propose d'utiliser une composition comprenant au moins un extrait de propolis de peuplier présentant au moins les caractéristiques suivantes :
- une teneur en flavones et flavonols supérieure ou égale à 5,5% en poids par rapport au poids total de matière sèche de l'extrait,
- une teneur en flavanones et dihydroflavanols supérieure ou égale à 5% en poids par rapport au poids total de matière sèche de l'extrait,
- une valeur antioxydante (ORAC) supérieure ou égale à 500 mmol TE/100g d'extrait sec,
- une teneur en polyphénols totaux supérieure ou égale à 30% en poids par rapport au poids total de matière sèche de l'extrait, pour son utilisation comme produit de santé pour prévenir et/ou limiter les effets secondaires des chimiothérapies chez l'être humain ou l'animal.

En particulier, l'invention vise une composition comprenant au moins un tel extrait de propolis, pour son application comme produit de santé, en particulier comme complément nutritionnel oral humain ou comme médicament pour prévenir et/ou limiter les effets secondaires des chimiothérapies. Préférentiellement, la composition est un Aliment Diététique Destiné à des Fins Médicales Spéciales (ADDFMS).

De façon surprenante, l'extrait de propolis particulier selon l'invention présente une bonne efficacité pour prévenir et/ou limiter les effets secondaires des chimiothérapies, sans limiter leur action au niveau des cellules cancéreuses.

L'invention est maintenant décrite en détails.

L'invention vise donc une composition comprenant au moins un extrait de propolis, ledit extrait de propolis de peuplier présentant au moins une des caractéristiques suivantes :
- une teneur en flavones et flavonols supérieure ou égale à 5,5% en poids par rapport au poids total de matière sèche de l'extrait,
- une teneur en flavanones et dihydroflavanols supérieure ou égale à 5% en poids par rapport au poids total de matière sèche de l'extrait,
- une valeur antioxydante (ORAC) supérieure ou égale à 500 mmol TE/100g d'extrait sec,
- une teneur en polyphénols totaux supérieure ou égale à 30% en poids par rapport au poids total de matière sèche de l'extrait, pour son utilisation pour prévenir et/ou limiter les effets secondaires des chimiothérapies chez l'être humain ou l'animal.

Par extrait de propolis on entend toute propolis récoltée transformée par un procédé d'extraction permettant d'enlever les impuretés présentes dans l'extrait brut et/ou de concentrer la propolis en un ou plusieurs de ses constituants.

L'extrait de propolis peut se présenter sous toute forme. Préférentiellement, il se présente sous forme de poudre.

L'extrait de propolis utile selon l'invention est un extrait de propolis de peuplier présentant au moins une des caractéristiques suivantes :
- une valeur antioxydante (ORAC) supérieure ou égale à 500 mmol TE/100g d'extrait sec,
- une teneur en flavones et flavonols supérieure ou égale à 5,5% en poids par rapport au poids total de matière sèche de l'extrait, les flavones pouvant être notamment représentés par chrysine et/ou apigenine, et les flavonols pouvant être notamment représentés par Kaempferol et/ou galangine.
- une teneur en flavanones et dihydroflavanols supérieure ou égale à 5% en poids par rapport au poids total de matière sèche de l'extrait, les flavanones pouvant être notamment représentés par pinocembrine, et les dihydroflavanols pouvant être notamment représentés par pinobanksine.

Selon une variante préférée l'extrait de propolis utile selon l'invention est un extrait de propolis présentant obligatoirement une valeur antioxydante (ORAC) supérieure ou égale à 500 mmol TE/100 g d'extrait. La valeur antioxydante d'un extrait de propolis selon l'invention est déterminée selon la méthode ORAC décrite par Ou et al. (2001), dans JAFC, 49 (10), 4619-4626.

Préférentiellement l'extrait de propolis présente également une teneur en flavones et flavonols supérieure ou égale à 5,5% en poids par rapport au poids total de matière sèche de l'extrait et/ou une teneur en flavanones et dihydroflavanols supérieure ou égale à 5% en poids par rapport au poids total de matière sèche de l'extrait.

Selon une deuxième variante l'extrait de propolis est un extrait présentant obligatoirement une teneur en flavones et flavonols supérieure ou égale à 5,5% en poids par rapport au poids total de matière sèche de l'extrait.

Préférentiellement l'extrait de propolis présente également une teneur en flavanones et dihydroflavanols supérieure ou égale à 5% en poids par rapport au poids total de matière sèche de l'extrait et/ou une valeur antioxydante (ORAC) supérieure ou égale à 500 mmol TE/100 g d'extrait sec.

Selon une troisième variante, l'extrait de propolis présente obligatoirement une teneur en flavanones et dihydroflavanols supérieure ou égale à 5% en poids par rapport au poids total de matière sèche de l'extrait.

Préférentiellement l'extrait de propolis présente également une teneur en flavones et flavonols supérieure ou égale à 5,5% en poids par rapport au poids total de matière sèche de l'extrait et/ou une valeur antioxydante (ORAC) supérieure ou égale à 500 mmol TE/100g d'extrait sec.

Quelle que soit la variante, l'extrait de propolis présente préférentiellement également une teneur en polyphénols totaux supérieure ou égale à 30% en poids de matière sèche de l'extrait.

La propolis utilisée peut provenir de toute origine botanique bien identifiée. Par exemple il peut s'agir de propolis de peuplier ou de propolis de *Baccharis* (propolis verte du Brésil), notamment de *Baccharis dracunculifolia.*

Très préférentiellement la composition comprend un extrait de propolis de peuplier. L'extrait présent dans la composition peut être obtenu par un procédé comprenant les étapes suivantes :
- extraction de propolis,
- concentration pour obtenir une valeur antioxydante (ORAC) supérieure ou égale à 500 mmol TE/100g d'extrait sec, et/ou une teneur en flavones et flavonols supérieure ou égale à 5,5% en poids par rapport au poids total de matière sèche de l'extrait et/ou une teneur en flavanones et dihydroflavanols supérieure ou égale à 5% en poids par rapport au poids total de matière sèche de l'extrait, et préférentiellement au moins 30% de polyphénols en poids de matière sèche de l'extrait.

L'extrait peut ensuite être reconcentré.

Les différentes étapes du procédé doivent être réalisées, sans détruire les principes actifs. Selon un mode de réalisation particulièrement adapté, l'extrait utilisé selon l'invention peut être obtenu par un procédé comprenant les étapes suivantes :
- macération de propolis brute dans une solution alcoolique, et
- concentration par évaporation.
- mise en poudre selon un procédé de mélange de la pâte de propolis avec les excipients en froid négatif pour préserver l'intégrité des principes actifs pendant ce mélange.

Le précédent procédé permet l'obtention d'une poudre de propolis très concentrée en principes actifs et dépourvue de cires et autres impuretés contenues dans la matière première. Les poudres de propolis du marché étant produites directement à partir d'un mélange de la matière première avec des excipients.

L'extrait de propolis obtenu présente au moins une des caractéristiques suivantes :
- une valeur antioxydante (ORAC) supérieure ou égale à 500 mmol TE/100g d'extrait sec,
- une teneur en flavones et flavonols supérieure ou égale à 5,5% en poids par rapport au poids total de matière sèche de l'extrait,
- une teneur en flavanones et dihydroflavanols supérieure ou égale à 5% en poids par rapport au poids total de matière sèche de l'extrait.

Très préférentiellement il comprend au moins 30% en poids de polyphénols totaux.

L'extrait peut ensuite être transformé en poudre.

L'extrait de propolis obtenu est incorporé dans une composition.

La composition comprenant l'extrait de propolis est utilisée comme produit de santé chez l'homme ou l'animal, en particulier comme complément nutritionnel oral humain ou comme médicament ou comme produit vétérinaire.

Préférentiellement, la composition selon l'invention est un complément nutritionnel en particulier un Aliment Diététique Destiné à des Fins Médicales Spéciales (ADDFMS).

La composition selon l'invention, en plus de l'extrait de propolis, peut contenir des protéines, des glucides, des lipides, des vitamines et/ou des minéraux, ajoutés en suppléments. Préférentiellement ces molécules sont ajoutées selon la règlementation en vigueur sur les ADDFMS.

La composition peut également contenir des excipients connus de l'homme de l'art, tels que du kaolin, de la poudre de caroube, de la silice ou du fibregum® ou d'autres agents texturants, fluidifiants, d'enrobage et de gastro-résistance connus dans le domaine pharmaceutique tels que de l'éthylcellulose ou de l'alginate sodique ou d'autres agents d'enrobage et de gastro-résistance.

Selon un mode de réalisation adapté, la composition se présente sous forme d'une poudre.

La composition selon l'invention, lorsqu'il s'agit d'un complément nutritionnel oral est administrée en plus et/ou en substitut des repas.

De façon préférée, la dose journalière de composition comprend entre 600 et 1200 mg d'extrait de poudre de propolis en poids de matière sèche. Préférentiellement, la dose journalière est répartie en 2 ou 3 prises.

La composition selon l'invention comprenant au moins un extrait de propolis selon l'invention est utilisée pour son application pour prévenir et/ou limiter les effets secondaires des chimiothérapies. Elle est préférentiellement utilisée avant l'administration d'agents de chimiothérapie pour préparer les cellules à être agressées par les agents de chimiothérapie. En particulier, la composition selon l'invention peut être utilisée pour préserver et/ou limiter la diminution des globules blancs, des globules rouges, des plaquettes sanguines ou des altérations hépatiques, rénales ou cardiaques consécutives aux injections d'agents de chimiothérapie.

En effet, l'injection d'agents de chimiothérapie provoque une diminution des globules blancs, des globules rouges et des plaquettes sanguines, et l'utilisation d'un extrait de propolis selon l'invention permet de limiter cette diminution.

En outre, la composition selon l'invention peut être utilisée pour préserver et/ou limiter les dégâts radicalaires causés par les agents de chimiothérapie sur des organes, en particulier sur le foie, les reins et/ou le coeur, notamment en stimulant la voie de signalisation Nrf2 impliquée dans la défense antioxydante endogène de la cellule.

Avantageusement, grâce à son action notamment sur la limitation de la diminution des globules blancs, des globules rouges et des plaquettes sanguines, et sur la limitation des dégâts radicalaires, la composition selon l'invention permet de prévenir et/ou lutter contre les nausées, chutes de cheveux, chutes d'ongles, états de fatigue, pertes de poids, défaillance hépatique, rénale et/ou cardiaque et déprimes des personnes sous traitement chimiothérapeutique.

L'invention est à présent illustrée par des exemples et des résultats d'essais.

### Exemple 1 : exemple d'extrait de propolis

La propolis utilisée, en particulier la propolis de peuplier, est obtenue par la mise en oeuvre de la technique des grilles qui permet d'obtenir une propolis avec des caractéristiques particulières adaptées à une utilisation médicale en comparaison d'une propolis obtenue par la technique de grattage. La méthode des grilles permet d'obtenir une propolis avec un taux de polyphénols plus important et un pourcentage de cire diminué.

Préférentiellement, le pourcentage de cire dans la propolis récoltée est inférieure à 21%, encore plus préférentiellement inférieur à 17%.

Une fois la propolis récoltée, elle est traitée par la mise en oeuvre d'un procédé d'extraction comprenant les étapes suivantes :
- la propolis est mélangée dans un extracteur avec de l'alcool suivant un ratio de 1/2,5 à 1/5 (w/v) pendant un laps de temps déterminé,
- le mélange subit ensuite une filtration afin de ne garder en solution liquide que les principes actifs de la propolis : les polyphénols,
- une dernière clarification par décantation gravitaire peut éventuellement être réalisée si nécessaire.

Ce procédé permet l'élaboration d'un extrait liquide chargé en principes actifs et dépourvu de cires et autres impuretés contenues dans la matière brute.

Cet extrait liquide est ensuite concentré en principes actifs par desalcoolisation, les paramètres devant être ajustés pour obtenir un extrait de propolis présentant les caractéristiques suivantes :
- une valeur antioxydante (ORAC) supérieure ou égale à 500 mmol TE/100g d'extrait sec,
- une teneur en flavones et flavonols supérieure ou égale à 5,5% en poids par rapport au poids total de matière sèche de l'extrait
- une teneur en flavanones et dihydroflavanols supérieure ou égale à 5% en poids par rapport au poids total de matière sèche de l'extrait, et
- préférentiellement une teneur en polyphénols totaux supérieure ou égale à 30% en poids par rapport au poids total de matière sèche de l'extrait.

On obtient alors un extrait mou très concentré en principes actifs qui peut être transformé en poudre.

Un exemple d'extrait de propolis obtenu par la mise en oeuvre de ce procédé est un extrait de propolis de peuplier sous forme de poudre, présentant les caractéristiques suivantes :
- une valeur antioxydante (ORAC) compris entre 500 et 750 mmolTE/100g d'extrait sec (valeur moyenne 698)
- une teneur en flavones et flavonols comprise entre 5.5% et 10% en poids par rapport au poids total de matière sèche de l'extrait (moyenne 8,6%)
- une teneur en flavanones et dihydroflavanols comprise entre 5% et 10% en poids par rapport au poids total de matière sèche de l'extrait (moyenne 7,1%)
- une teneur en polyphénols totaux comprise entre 30% et 50% (moyenne 38,7%).

### Exemple 2 : exemple de composition

Un exemple de composition utile selon l'invention comprend :
- 65% de l'extrait de l'exemple 1, et
- 10% de poudre de caroube, et/ou,
- 10% de silice, et/ou,
- 15 % d'au moins un autre excipient (stéarate de magnésium).

### Exemple 3 : exemple de composition

Un exemple de composition utile selon l'invention comprend :
- 75% de l'extrait de l'exemple 1, et
- 5% de poudre de caroube, et/ou,
- 10% de silice, et/ou,
- 10 % d'au moins un autre excipient (stéarate de magnésium).

### Exemple 4 : exemple de composition

Un exemple de composition utile selon l'invention comprend :
- 75% de l'extrait de l'exemple 1, et
- 25% de kaolin,

### Evaluation des différences physico-chimiques et biologiques de différents extraits de propolis

Les caractéristiques de différentes poudres de propolis ont été testées :
- Poudre selon l'exemple 1
- Poudres de l'art antérieur (poudres de propolis du commerce) : la poudre 1 provient d'Aagaard, la poudre 2 d'Aprolis et la poudre 3 d'Apimab.

Les dosages de polyphénols totaux et des 2 sous fractions flavonoïdes ont été effectués par spectrophotométrie selon les méthodes décrites et validées par Popova et al. (2004), 15 : 235-240 dans Phytochemical Analysis.

La valeur antioxydante est déterminée selon la méthode ORAC décrite par Ou et al. (2001), dans JAFC, 49 (10), 4619-4626.

Les résultats sont présentés dans le tableau ci-après :

| | Polyphénols totaux (g/100 g) | Flavones et flavonols (g/100 g) | Flavanones et dihydroflavanols (g/100 g) | ORAC (mmol TE/100 g) |
|---|---|---|---|---|
| poudre exemple 1 | 38.7 | 8.3 | 7.1 | 698 |
| Poudre 1 | 14.1 | 1.5 | 4.7 | 98 |
| Poudre 2 | 34.4 | 7.2 | 7.2 | 480 |
| Poudre 3 | 12.3 | 1.9 | 3.5 | 177 |

### Evaluation in vitro de l'effet de la propolis sur les effets secondaires des chimiothérapies

### Mesure de la réversion de la toxicité d'agent chimiothérapique.

Des hépatocytes primaires de rats ont été prélevés et ensemencés en plaque de 96 puits. 24H après l'ensemencement, les hépatocytes ont été traités avec différentes doses de propolis (exemple 1) pendant 24h. A la fin des 24h de prétraitement, la propolis est retirée et les hépatocytes sont traités avec des composés de chimiothérapie cytotoxiques (tamoxifene et cisplatine).

Le pourcentage de cellules hépatiques cytolysées après un prétraitement à différentes concentrations de propolis puis soumis à une concentration cytotoxique de tamoxifène (25 µM) a été évalué et présenté dans le tableau ci-dessous :

| **Heures** | Non Traité | propolis 1 µg/ml | propolis 3 µg/ml | propolis 10 µg/ml |
|---|---|---|---|---|
| 0 | 4,09 | 3,54 | 4,03 | 4,04 |
| 2 | 4,04 | 3,61 | 4,08 | 4,20 |
| 4 | 4,44 | 4,02 | 4,41 | 4,19 |
| 6 | 5,67 | 5,54 | 5,21 | 4,65 |
| 8 | 7,03 | 6,92 | 5,68 | 4,80 |
| 10 | 8,75 | 8,29 | 6,32 | 4,91 |
| 12 | 10,22 | 9,78 | 6,78 | 4,96 |
| 14 | 11,71 | 10,82 | 7,11 | 5,01 |
| 16 | 12,76 | 11,89 | 7,46 | 5,03 |
| 18 | 13,85 | 12,89 | 7,68 | 4,89 |
| 20 | 14,46 | 14,16 | 7,89 | 4,85 |
| 22 | 14,94 | 14,61 | 8,21 | 4,90 |
| 24 | 15,47 | 15,28 | 8,26 | 4,98 |
| 26 | 15,87 | 15,85 | 8,54 | 5,01 |
| 28 | 16,41 | 16,09 | 8,80 | 5,13 |
| 30 | 16,60 | 16,51 | 9,00 | 4,87 |
| 32 | 17,30 | 16,90 | 9,20 | 4,68 |
| 34 | 17,94 | 17,46 | 9,20 | 4,70 |
| 36 | 18,22 | 17,42 | 9,44 | 4,45 |
| 38 | 18,67 | 18,08 | 9,74 | 4,54 |

De même, le pourcentage de cellules hépatiques cytolysées après un prétraitement à différentes concentrations de propolis puis soumis à une concentration cytotoxique de cisplatine (10µM) est présenté dans le tableau ci-après :

| **heures** | **Non Traité** | **propolis 0,1 µg/ml** | **propolis 0,3 µg/ml** | **propolis 1 µg/ml** | **propolis 10 µg/ml** |
|---|---|---|---|---|---|
| 0 | 1,31 | 1,83 | 1,78 | 2,27 | 2,10 |
| 2 | 1,11 | 1,43 | 1,01 | 1,77 | 1,67 |
| 4 | 0,99 | 1,21 | 0,67 | 1,51 | 1,37 |
| 6 | 0,95 | 1,20 | 0,60 | 1,52 | 1,29 |
| 8 | 0,92 | 1,06 | 0,57 | 1,43 | 1,28 |
| 10 | 0,89 | 1,01 | 0,48 | 1,39 | 1,14 |
| 12 | 0,85 | 1,02 | 0,57 | 1,31 | 1,06 |
| 14 | 0,86 | 1,09 | 0,69 | 1,39 | 1,16 |
| 16 | 1,15 | 1,18 | 0,92 | 1,45 | 1,28 |
| 18 | 1,42 | 1,48 | 1,08 | 1,52 | 1,29 |
| 20 | 1,90 | 1,77 | 1,52 | 2,07 | 1,74 |
| 22 | 2,82 | 2,30 | 1,83 | 2,63 | 1,87 |
| 24 | 4,29 | 3,33 | 2,98 | 3,45 | 2,30 |
| 26 | 5,82 | 4,59 | 3,96 | 4,14 | 3,33 |
| 28 | 7,68 | 6,10 | 4,81 | 5,12 | 3,98 |
| 30 | 9,70 | 7,53 | 6,44 | 6,83 | 5,58 |
| 32 | 12,48 | 9,05 | 7,98 | 8,08 | 6,27 |
| 34 | 15,01 | 11,10 | 9,25 | 9,71 | 7,66 |
| 36 | 16,84 | 12,10 | 10,28 | 10,63 | 8,37 |
| 38 | 18,49 | 13,46 | 11,43 | 11,43 | 9,22 |
| 40 | 20,46 | 15,04 | 11,93 | 12,96 | 9,71 |
| 42 | 21,74 | 15,78 | 12,68 | 14,38 | 11,02 |
| 44 | 23,61 | 17,33 | 13,59 | 14,53 | 11,10 |
| 46 | 25,68 | 18,65 | 14,18 | 15,71 | 12,14 |
| 48 | 27,07 | 19,45 | 14,71 | 16,95 | 12,34 |
| 50 | 28,48 | 20,76 | 15,36 | 16,89 | 12,94 |
| 52 | 30,37 | 22,09 | 16,65 | 17,26 | 13,28 |

Le tamoxifène et le cisplatine entrainent une cytolyse dose-dépendante des hépatocytes, où l'augmentation de la dose induit une augmentation des cellules cytolysées avec une cytolyse complète entre 6 et 48h pour la dose la plus élevée.

On constate qu'un prétraitement avec un extrait de propolis selon l'invention présente un effet protecteur hépatocytaire contre le tamoxifène. Il confère une protection contre le traitement de chimiothérapie comparable à un composé hépato protecteur de référence connu : l'oltipraz.

On constate également que l'extrait de propolis selon l'invention est capable de diminuer les effets toxiques du cisplatine.

### Evaluation in vivo de l'effet de la propolis sur les effets secondaires des chimiothérapies

Des rats wistars d'environ 200g ont été utilisés lors de cette expérience in vivo, à raison de 8 animaux par groupe. Les animaux ont reçu oralement soit le véhicule (gr contrôle) soit de la propolis (exemple 1) à 12.5mg de poudre/Kg de poids corporel pendant 5 jours consécutifs avant l'injection d'un agent de chimiothérapie (épirubicine, cyclophosphamide, 5-FU et taxotère), soit les agents de chimiothérapie seuls. Les animaux sont sacrifiés 21 jours après l'injection de l'agent de chimiothérapie. Le poids et les différents paramètres de la formule sanguine seront suivis chez ces animaux aux jours J+1, J+6 et J+14 et J+21 post injection de chimiothérapie. Les teneurs en antioxydants endogènes (glutathion =GSH) et les niveaux de peroxydation lipidiques (MDA) hépatocytaire seront évalués à J+21.

**Evolution du poids des animaux des différents groupes.**

| | J-5 | J+1 | J+6 | J+14 | J+21 | Variation entre j+1 et j+21 |
|---|---|---|---|---|---|---|
| Gr contrôle | 151.6 | 164.5 | | | | |
| Gr epirubicine | 150.2 | 156.1 | 156.1 | 149.5 | 155.2 | -0.9 |
| Gr epirubicine + propolis | 157 | 163.5 | 168.6 | 171.5 | 178.4 | + 14.9 |
| Gr cyclophosphamide | 160.4 | 167.2 | 157.7 | 164 | 152.3 | -14.9 |
| Gr cyclophosphamide + propolis | 155.6 | 162.6 | 165.6 | 169.2 | 181.4 | + 18.8 |
| Gr 5-FU | 153.5 | 157.6 | 153.7 | 147.9 | 143.7 | -13.9 |
| Gr 5-FU + propolis | 153.6 | 158.6 | 163.4 | 167.7 | 169 | + 10.4 |
| Gr taxotère | 151.6 | 156.9 | 164.6 | 170.7 | 175.5 | + 18.6 |
| Gr taxotère+ propolis | 151.7 | 158.7 | 165.6 | 173.5 | 180.4 | + 21.7 |

On constate que les animaux traités avec les agents de chimiothérapie voient leur poids décroître rapidement après l'injection (sauf pour le taxotère). Le prétraitement avec l'extrait de propolis selon l'invention (exemple 1) rétablit la courbe de croissance pondérale des animaux traités.

**Evolution des globules rouges des animaux des différents groupes.**

| | J-5 | J+1 | J+6 | J+14 | J+21 | Variation entre j+1 et j+21 |
|---|---|---|---|---|---|---|
| Gr contrôle | 6.6 | 6.2 | | | | |
| Gr epirubicine | 6.73 | 5.95 | 5.04 | 4.72 | 3.07 | - 48 % |
| Gr epirubicine + propolis | 6.52 | 6.03 | 5.83 | 5.33 | 4.81 | - 20 % |
| Gr cyclophosphamide | 6.51 | 5.94 | 4.23 | 3.80 | 3.22 | - 46 % |
| Gr cyclophosphamide + propolis | 6.69 | 6.24 | 5.84 | 5.28 | 4.60 | - 26% |
| Gr 5-FU | 6.51 | 6.56 | 4.19 | 3.05 | 2.94 | - 55 % |
| Gr 5-FU + propolis | 6.67 | 6.40 | 5.99 | 5.15 | 5.31 | - 17% |
| Gr taxotère | 6.39 | 6.23 | 5.18 | 4.18 | 2.95 | - 52% |
| Gr taxotère+ propolis | 6.47 | 6.23 | 5.95 | 4.92 | 4.61 | - 26 % |

Il est connu que l'un des effets secondaires des agents de chimiothérapie est leur capacité à détruire les lignées sanguines conduisant souvent à une aplasie des individus. Les résultats confirment que l'injection des agents entraîne une diminution très nette et significative des globules rouges - 48, 45, 55 et 52 % au 21j post injection pour l'épirubicine, cyclophosphamide, 5-FU et taxotère, respectivement. Quel que soit l'agent, l'extrait de propolis selon l'invention entraîne une réduction significative de moitié de la perte de globules rouges.

**Evolution des globules blancs des animaux des différents groupes.**

| | J-5 | J+1 | J+6 | J+14 | J+21 | Variation entre j-5 et j+21 |
|---|---|---|---|---|---|---|
| Gr contrôle | 6.83 | 8.38 | | | | |
| Gr epirubicine | 6.79 | 3.55 | 2.32 | 12.54 | 3.63 | - 46 % |
| Gr epirubicine + propolis | 6.65 | 5.76 | 4.71 | 8.40 | 5.26 | - 21 % |
| Gr cyclophosphamide | 6.30 | 3.33 | 2.96 | 10.20 | 3.19 | - 49% |
| Gr cyclophosphamide + propolis | 6.41 | 6.01 | 5.18 | 9.36 | 4.63 | - 28 % |
| Gr 5-FU | 6.49 | 3.91 | 2.74 | 10.82 | 3.86 | - 40% |
| Gr 5-FU + propolis | 6.65 | 5.77 | 5.18 | 9.53 | 5.67 | -15% |
| Gr taxotère | 6.44 | 4.41 | 4.29 | 10.18 | 3.13 | -51% |
| Gr taxotère+ propolis | 6.50 | 6 | 5.34 | 9.28 | 4.26 | - 33% |

Concernant les globules blancs, les agents de chimiothérapie entraînent une chute au 6^{ème} jour, suivie par une remontée très nette au 14^{ème} jour, elle-même suivie d'une nouvelle chute au 21^{ème} jour. Globalement le prétraitement avec l'extrait de propolis selon l'invention limite la chute de globules blancs au 6^{ème} jour et au 21^{ème} jour.

**Variation de la teneur en créatinine plasmatique des animaux des différents groupes.**

| | J-5 | J+1 | J+21 | Variation entre j-5 et j+21 |
|---|---|---|---|---|
| Gr contrôle | 6.17 | 6.33 | | |
| Gr epirubicine | 7.13 | 10.89 | 11.39 | + 60% |
| Gr epirubicine + propolis | 7 | 9.3 | 9.4 | + 34% |
| Gr cyclophosphamide | 6.38 | 7.25 | 10.14 | + 59% |
| Gr cyclophosphamide + propolis | 6.38 | 6 | 6.13 | - 4% |
| Gr 5-FU | 7.25 | 9 | 10.19 | + 41% |
| Gr 5-FU + propolis | 8.25 | 7.29 | 8.81 | + 7% |
| Gr taxotère | 7.60 | 8.91 | 11.53 | + 52 % |
| Gr taxotère+ propolis | 7.81 | 7.80 | 8.90 | + 14% |

Le dosage de la créatinine plasmatique, reflet de la fonctionnalité des reins, montre que l'injection des différents agents de chimiothérapie entraîne une élévation plus ou moins marquée de ce paramètre à J+1 et J+21 traduisant une atteinte rénale. Le prétraitement avec l'extrait de propolis selon l'invention réduit voire restaure à son niveau basal la créatinine plasmatique.

**Variation de la teneur en glutathion plasmatique des animaux des différents groupes.**

| | J-5 | J+21 | Variation entre j-5 et j+21 |
|---|---|---|---|
| Gr contrôle | 20.92 | | |
| Gr epirubicine | 23.86 | 12.83 | - 46% |
| Gr epirubicine + propolis | 22.80 | 20.35 | -11 % |
| Gr cyclophosphamide | 26.39 | 23.81 | -10 % |
| Gr cyclophosphamide + propolis | 22.79 | 23.20 | + 2% |
| Gr 5-FU | 22.14 | 17.65 | -20% |
| Gr 5-FU + propolis | 22.71 | 24.67 | + 8% |
| Gr taxotère | 22.88 | 16.83 | -26% |
| Gr taxotère+ propolis | 23.61 | 23.61 | 0 |

**Variation de la teneur en MDA plasmatique des animaux des différents groupes.**

| | J-5 | J+21 | Variation entre j-5 et j+21 |
|---|---|---|---|
| Gr contrôle | 46.08 | | |
| Gr epirubicine | 46.61 | 85.45 | + 83 % |
| Gr epirubicine + propolis | 45.91 | 58.62 | + 28% |
| Gr cyclophosphamide | 43.78 | 79.10 | +48% |
| Gr cyclophosphamide + propolis | 44.49 | 52.26 | + 17% |
| Gr 5-FU | 44.49 | 62.85 | +41% |
| Gr 5-FU + propolis | 41.66 | 47.31 | + 13% |
| Gr taxotère | 45.90 | 86.16 | + 88 % |
| Gr taxotère+ propolis | 43.07 | 63.56 | + 47% |

Les agents de chimiothérapie ont également un impact sur le principal antioxydant intracellulaire : le glutathion (GSH) et sur les dégâts oxydatifs au travers la formation de diènes-conjugués qui est le reflet de la peroxydation lipidique des membranes cellulaires (le MDA) au niveau plasmatique. Ces deux mesures sont complémentaires puisque normalement un taux correct de GSH permet de maintenir un niveau relativement bas de MDA.

On constate que l'injection d'épirubicine, de 5-FU et de taxotère entraîne une baisse significative très nette du taux de GSH plasmatique au 21^{ème} jour (et un peu moins nette pour le cyclophosphamide). Parallèlement, les taux de MDA sont multipliés par 2 pour tous les agents de chimiothérapie au 21^{ème} jour. Le prétraitement avec l'extrait de propolis selon l'invention (exemple 1) permet de maintenir à son niveau basal le taux de GSH quel que soit l'agent de chimiothérapie utilisé. Dans le même temps, les niveaux de MDA des animaux prétraités avec l'extrait de propolis selon l'invention (exemple 1) sont significativement réduits par rapport aux groupes traités avec les agents de chimiothérapie seuls.

## Revendications

1. Composition comprenant au moins un extrait de propolis de peuplier, ledit extrait de propolis présentant au moins-les caractéristiques suivantes :
- une valeur antioxydante (ORAC) supérieure ou égale à 500 mmol TE/100g d'extrait sec,
- une teneur en flavones et flavonols supérieure ou égale à 5,5% en poids par rapport au poids total de matière sèche de l'extrait,
- une teneur en flavanones et dihydroflavanols supérieure ou égale à 5% en poids par rapport au poids total de matière sèche du produit, et
- une teneur en polyphénols totaux supérieure ou égale à 30% en poids par rapport au poids total de matière sèche de l'extrait.
pour son utilisation pour prévenir et/ou limiter les effets secondaires des chimiothérapies chez l'être humain ou l'animal.

2. Composition pour une utilisation selon l'une des précédentes revendications comme produit de santé.

3. Composition pour son utilisation selon l'une des précédentes revendications, comme complément nutritionnel oral humain, comme médicament ou comme produit vétérinaire.

4. Composition pour une utilisation selon l'une des précédentes revendications, comme Aliment Diététique Destiné à des Fins Médicales Spéciales.

5. Composition pour une utilisation selon l'une des précédentes revendications, pour limiter et/ou préserver de la diminution des globules blancs, des globules rouges et des plaquettes sanguines consécutives aux injections d'agents de chimiothérapie.

6. Composition pour une utilisation selon l'une des précédentes revendications, pour préserver et/ou limiter les dégâts radicalaires causés par les agents de chimiothérapie sur des organes.

7. Composition pour une utilisation selon l'une des précédentes revendications, pour préserver et/ou limiter les dégâts radicalaires causés par les agents de chimiothérapie sur le foie, les reins et/ou le coeur.

8. Composition pour une utilisation selon l'une des précédentes revendications, pour prévenir et/ou lutter contre les nausées, chutes de cheveux, chutes d'ongles, états de fatigue, pertes de poids et déprimes des personnes sous traitement chimiothérapeutique.

9. Composition pour une utilisation selon l'une des précédentes revendications, **caractérisée en ce qu'**elle comprend également des protéines, des glucides, des lipides, des vitamines et/ou des minéraux.

10. Composition pour une utilisation selon l'une des précédentes revendications, **caractérisée en ce qu'**il se présente sous forme d'une poudre.

11. Composition pour une utilisation selon l'une des précédentes revendications, **caractérisée en ce qu'**elle est administrée en plus et/ou en substitut des repas.

## Patentansprüche

1. Zusammensetzung mit wenigstens einem Extrakt aus Bienenharz von Pappeln, wobei der Extrakt aus Bienenharz wenigstens die folgenden Eigenschaften aufweist:
- einen antioxidativer Wert (ORAC) größer gleich 500 mmol TE/100g an trockenem Extrakt,
- einen Gehalt an Flavonen und Flovonolen größer gleich 5,5 Gew.-% bezüglich des gesamten Trockengewichts des Extrakts,
- einen Gehalt an Flavanonen und Dihydroflavanolen größer gleich 5 Gew.-% bezüglich des gesamten Trockengewichts des Produkts, und
- einen Gehalt an allen Polyphenolen größer gleich 30 Gew.-% bezüglich des gesamten Trockengewichts des Extrakts,
zu deren Verwendung zur Prävention und/oder Begrenzung von Nebenwirkungen von Chemotherapien bei Mensch oder Tier.

2. Zusammensetzung zu deren Verwendung nach einem der vorhergehenden Ansprüche als Gesundheitsprodukt.

3. Zusammensetzung zu deren Verwendung nach einem der vorhergehenden Ansprüche als orales Nahrungsergänzungsmittel für Menschen, als Medikament oder Veterinärprodukt.

4. Zusammensetzung zu deren Verwendung nach einem der vorhergehenden Ansprüche als diätetisches Lebensmittel, das zu speziellen medizinischen Zwecken bestimmt ist.

5. Zusammensetzung zu deren Verwendung nach einem der vorhergehenden Ansprüche, um die Verringerung der weißen Blutkörperchen, der roten Blutkörperchen oder der Blutplättchen infolge von Injektionen von chemotherapeutischen Wirkstoffen zu begrenzen und/oder zu verhindern.

6. Zusammensetzung zu deren Verwendung nach einem der vorhergehenden Ansprüche, um durch Radikale an den Organen hervorgerufene Schäden, die durch die chemotherapeutischen Wirkstoffe verursacht werden, zu verhindern und/oder zu begrenzen.

7. Zusammensetzung zu deren Verwendung nach einem der vorhergehenden Ansprüche, um die durch Radikale an der Leber, den Nieren und/oder dem Herz hervorgerufene Schäden, die durch die chemotherapeutischen Wirkstoffe verursacht werden, zu verhindern und/oder zu begrenzen.

8. Zusammensetzung zu deren Verwendung nach einem der vorhergehenden Ansprüche zur Prävention und/oder zur Bekämpfung von Übelkeit, Haarausfall, Nagelausfall, Ermüdungszuständen, Gewichtsverlusten und depressiven Stimmungen von Personen in chemotherapeutischer Behandlung.

9. Zusammensetzung zu deren Verwendung nach einem der vorhergehenden Ansprüche, **dadurch gekennzeichnet, dass** diese auch Proteine, Kohlenhydrate, Lipide, Vitamine und/oder Mineralstoffe enthält.

10. Zusammensetzung zu deren Verwendung nach einem der vorhergehenden Ansprüche, **dadurch gekennzeichnet, dass** diese in Gestalt eines Pulvers vorliegt.

11. Zusammensetzung zu deren Verwendung nach einem der vorhergehenden Ansprüche, **dadurch gekennzeichnet, dass** diese in Ergänzung zu und/oder als Ersatz von Mahlzeiten verabreicht wird.

## Claims

1. A composition comprising at least one extract of poplar propolis, said extract of propolis having at least the following characteristics:
- an antioxidant value (ORAC) greater than or equal to 500 m/mol TE/100 g of dry extract,
- a flavone and flavonol content greater than or equal to 5.5% by weight with respect to the total weight of solids of the extract,
- a flavanone and dihydroflavanol content greater than or equal to 5% by weight with respect to the total weight of solids of the product, and
- a total polyphenol content greater than or equal to 30% by weight with respect to the total weight of solids of the extract,
for use in preventing and/or limiting the side effects of chemotherapy in humans or animals.

2. A composition for use according to one of the preceding claims as a health product.

3. A composition for use according to either of the preceding claims, as a human oral food supplement, as a medication or as a veterinary product.

4. A composition for use according to any of the preceding claims, as a Dietetic Food for Special Medical Purposes.

5. A composition for use according to any of the preceding claims, for limiting and/or protecting from a reduction in white corpuscles, red corpuscles and blood platelets following injection of chemotherapy agents.

6. A composition for use according to any of the preceding claims, for protecting from and/or limiting radical damage caused to organs by chemotherapy agents.

7. A composition for use according to any of the preceding claims, for protecting from and/or limiting radical damage caused by chemotherapy agents to the liver, kidneys and/or heart.

8. A composition for use according to any of the preceding claims, for preventing and/or combating nausea, hair loss, nail loss, fatigue states, loss of weight and depression in persons under chemotherapy treatment.

9. A composition for use according to any of the preceding claims, **characterised in that** it also comprises proteins, carbohydrates, lipids, vitamins and/or minerals.

10. A composition for use according to any of the preceding claims, **characterised in that** it is in the form of a powder.

11. A composition for use according to any of the preceding claims, **characterised in that** it is administered in addition to and/or in substitution for meals.
